# EUROPEAN PATENT APPLICATION

(11) **EP 3 505 063 A1**
(43) Date of publication of application: **03.07.2019**
(21) Application number: 17210632.0
(22) Date of filing: 27.12.2017
(51) Int. Cl.: A61B 5/11, A61B 5/22, A61B 5/00, A63B 23/16

(54) **DUAL HANDGRIP MEASUREMENT DEVICE AND METHOD OF UTILIZING A DUAL HANDGRIP MEASUREMENT DEVICE**

(71) Applicant: Nokia Technologies Oy, 02610 Espoo (FI)
(72) Inventor: LINDHOLM, Harri, 00320 Helsinki (FI); PELLIKKA, Jarkko, 00220 Helsinki (FI); MÜLLER, Kiti, 00430 Helsinki (FI); WOLDEGEBRIEL, Michael, 02610 Espoo (FI); REMES, Jukka, 02610 Espoo (FI)
(74) Representative: Nokia EPO representatives

(57) **Abstract**

A dual handgrip measurement device and an associated method of utilizing the dual handgrip measurement device are provided. The dual handgrip measurement device includes first and second handgrips configured to be gripped by left and right hands of a user, respectively. The dual handgrip measurement device includes first and second force sensors associated with and configured to separately detect grip forces applied to the first and second handgrips, respectively. The dual handgrip measurement device is configured to detect at least one of a linear force along the longitudinal axis, such as a compressive force or a tensile force, or a rotational force between the first and second handgrips.

## Description

### TECHNICAL FIELD

An example embodiment relates generally to a device and method for measuring the forces applied by a user and, more particularly, to a dual handgrip measurement device and associated method for utilizing a dual handgrip measurement device for concurrently detecting the forces applied by the left and right hands of a user.

### BACKGROUND

A user may produce a grip force by squeezing a handgrip with their hand. A grip force of a user may be indicative of a number of health conditions. In this regard, studies have shown grip forces to be an indicator of poor health and premature aging. By way of a more particular example, a decrease in the grip force and/or an inability to maintain and repeat the grip force may be indicative of various musculoskeletal disorders, such as epicondylitis (also known as tennis elbow) or carpal tunnel syndrome; upper extremity trauma; peripheral nerve dysfunction, such as trauma-based or due to sensor or motor neuropathies that develop over time in conjunction with, for example, diabetes or renal disease; or brain level dysfunction, such as due to a stroke, Parkinson's disease or other memory disorders effecting, in particular, frontal lobe functions. A low grip strength may also be indicative of an increased likelihood of developing further functional limitations in daily living that may extend to other performance areas than bimanual functions.

A handheld dynamometer is utilized to measure the grip strength of a user. Handheld dynamometers are generally held by a user in one hand. The user then endeavors to close their fingers to form a first. In conjunction with attempting to close their fingers to form a fist, the user must overcome a force, such as a spring force, a hydraulic force or the like, provided by the handheld dynamometer. By measuring the grip force with the handheld dynamometer, either on a single occasion or repeatedly over the course of time, information is collected regarding the health of the user. This information may then be utilized in conjunction with the diagnosis or tracking of a condition and/or the monitor the user's rehabilitation efforts.

### BRIEF SUMMARY

A dual handgrip measurement device and an associated method of utilizing the dual handgrip measurement device are provided in accordance with an example embodiment. The dual handgrip measurement device permits grip forces of both the left and right hands of a user to be concurrently measured, thereby increasing the efficiency with which the measurements may be obtained relative to a handheld dynamometer that measures the grip forces of a single hand at any one time. The dual handgrip measurement device is configured to obtain additional and different measurements than the measurements that a handheld dynamometer that interacts with a single hand is able to measure. For example, the dual handgrip measurement device is configured to measure the linear force, such as the compressive force or the tensile force, applied by the left and right hands of the user and/or a rotational force between the left and right hands of the user. As such, the dual handgrip measurement device and method of example embodiment collect additional and different measurements that provide more information regarding the health and well-being of a user, thereby facilitating the diagnosis, rehabilitation or other treatment of the user.

In an example embodiment, a dual handgrip measurement device is provided that includes first and second handgrips configured to be gripped by left and right hands of a user, respectively. The dual handgrip measurement device includes first and second force sensors associated with and configured to separately detect grip forces applied to the first and second handgrips, respectively. The dual handgrip measurement device is configured to detect at least one of a linear force along a longitudinal axis that extends through the dual handgrip measurement device, such as a compressive force or a tensile force, or a rotational force between the first and second handgrips.

The dual handgrip measurement device of an example embodiment includes a third force sensor configured to detect the linear force including at least one of a compressive force or a tensile force along the longitudinal axis. In an example embodiment, the first and second handgrips are structurally joined together. At least one of the first and second force sensors of an example embodiment includes an inner force sensor located interior of and an outer force sensor located exterior of a respective hand. The dual handgrip measurement device of an example embodiment includes a user interface configured to provide information to the user regarding interaction with the dual handgrip measurement device. The user interface of this example embodiment may be configured to provide instructions to the user regarding a sequence of interactions between the user and the dual handgrip measurement device. The first and second force sensors of an example embodiment are configured to detect the grip forces concurrent with detection of at least one of the linear force or the rotational force. The dual handgrip measurement device of an example embodiment includes at least one processor configured to determine the difference between the grip forces detected by the first and second force sensors. The dual handgrip measurement device of an example embodiment includes at least one memory configured to store a representation of forces detected by the first and second force sensors.

In another example embodiment, a method of measuring grip forces of a user utilizing a dual handgrip measurement device includes providing the dual handgrip measurement device including first and second handgrips configured to be gripped by the left and right hands of a user, respectively. The method includes separately detecting grip forces applied to the first and second handgrips with first and second force sensors associated with the first and second handgrips, respectively. The method additionally includes detecting at least one of a linear force along a longitudinal axis that extends through a dual handgrip measurement device, such as a compressive force or a tensile force, or a rotational force between the first and second handgrips.

The method of an example embodiment includes providing information to the user via a user interface of the dual handgrip measurement device regarding interaction by the user with the dual handgrip measurement device. In this example embodiment, the method may provide information by providing instructions to the user regarding a sequence of interactions between the user and the dual handgrip measurement device. In an example embodiment, the grip forces are detected by the first and second force sensors concurrent with detection of at least one of the linear force or the rotational force. The method of an example embodiment determines a difference between the grip forces detected by the first and second force sensors. The method of another example embodiment includes causing a representation of forces detected by the first and second force sensors to be stored.

In a further example embodiment, a dual handgrip measurement device is provided that includes first and second handgrips configured to be gripped by left and right hands of a user, respectively. The dual handgrip measurement device includes means, such as first and second force sensors, for separately detecting grip forces applied to the first and second handgrips. The dual handgrip measurement device includes means for detecting at least one of a linear force along a longitudinal axis that extends through the dual handgrip measurement device, such as a compressive force or a tensile force, or a rotational force between the first and second handgrips.

The means for detecting the linear force includes, in one example embodiment, a third force sensor configured to detect the linear force including at least one of a compressive force or a tensile force along the longitudinal axis. In an example embodiment, the first and second handgrips are structurally joined together. The means for detecting the grip forces applied to a respective handgrip includes, in an example embodiment, an inner force sensor located interior of and an outer force sensor located exterior of a respective hand. The dual handgrip measurement device of an example embodiment includes means, such as a user interface, for providing information to the user regarding interaction with the dual handgrip measurement device, such as by providing instructions to the user regarding a sequence of interactions between the user and the dual handgrip measurement device. The grip forces may be detected concurrent with detection of at least one of the linear force or the rotational force. The dual handgrip measurement device of an example embodiment includes means for determining the difference between the grip forces. The dual handgrip measurement device of an example embodiment includes means for storing a representation of the grip forces.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
Figure 1 is a perspective view of a dual handgrip measurement device in accordance with an example embodiment to the present disclosure;
Figure 2 is a perspective view of the dual handgrip measurement device of Figure 1 being grasped by the left and right hands of a user;
Figure 3 is a cross-sectional representation of the dual handgrip measurement device in accordance with an example embodiment to the present disclosure;
Figure 4 is a block diagram of a dual handgrip measurement device of an example embodiment to the present disclosure;
Figure 5 is a flowchart of the operations performed utilizing a dual handgrip measurement device of an example embodiment to the present disclosure;
Figure 6 is a plan view of a dual handgrip measurement device retained by a fixture to permit single handed operation of the dual handgrip measurement device in accordance with an example embodiment of the present disclosure; and
Figure 7 is a plan view of a dual handgrip measurement device that is placed upon a support surface such that the longitudinal axis defined by the first and second handgrips extends substantially vertically in accordance with an example embodiment of the present disclosure.

### DETAILED DESCRIPTION

Some embodiments of the present invention will now be described more fully hereinafter with reference to the accompanying drawings, in which some, but not all, embodiments of the invention are shown. Indeed, various embodiments of the invention may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present invention. Thus, use of any such terms should not be taken to limit the spirit and scope of embodiments of the present invention.

Additionally, as used herein, the term 'circuitry' refers to (a) hardware-only circuit implementations (e.g., implementations in analog circuitry and/or digital circuitry); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that require software or firmware for operation even if the software or firmware is not physically present. This definition of 'circuitry' applies to all uses of this term herein, including in any claims. As a further example, as used herein, the term 'circuitry' also includes an implementation comprising one or more processors and/or portion(s) thereof and accompanying software and/or firmware. As another example, the term 'circuitry' as used herein also includes, for example, a baseband integrated circuit or applications processor integrated circuit for a mobile phone or a similar integrated circuit in a server, a cellular network device, other network device, field programmable gate array, and/or other computing device.

As defined herein, a "computer-readable storage medium," which refers to a physical storage medium (e.g., volatile or non-volatile memory device), may be differentiated from a "computer-readable transmission medium," which refers to an electromagnetic signal.

In an example embodiment, a dual handgrip measurement device is provided that includes first and second handgrips configured to be gripped by left and right hands of the user, respectively. The first and second handgrips define a longitudinal axis therethrough. The dual handgrip measurement device is configured to separately detect grip forces applied to the first and second handgrips. The dual handgrip measurement device is further configured to detect at least one of a linear force, such as a compressive force or a tensile force, along a longitudinal axis that extends though the dual handgrip measurement device or a rotational force between the first and second handgrips, such as about the longitudinal axis. As such, the dual handgrip measurement device is configured to detect a plurality of different types of forces applied by the hands of a user, thereby providing more information regarding the health and well-being of a user in order to facilitate the diagnosis, rehabilitation or other treatment of the user.

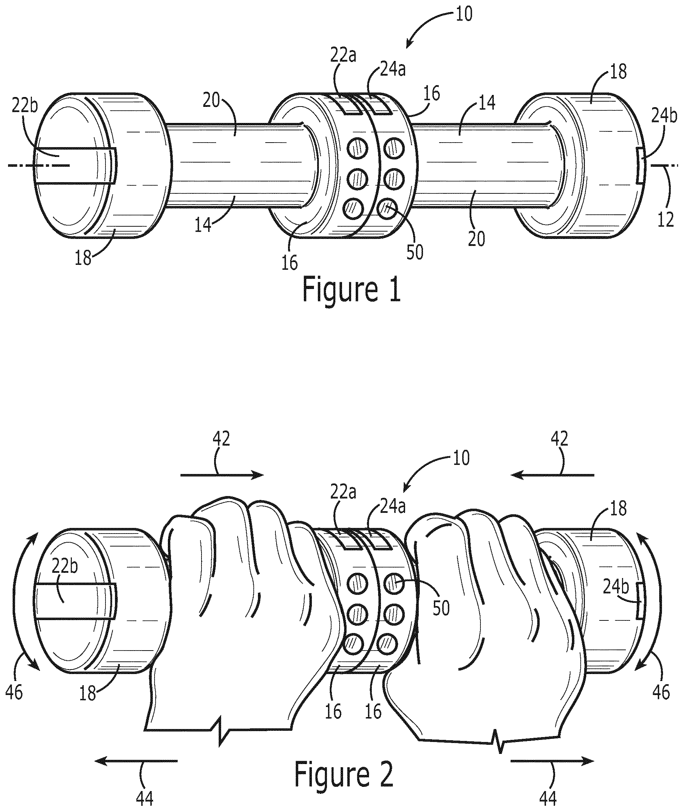

Referring now to Figure 1, a dual handgrip measurement device 10 is illustrated in accordance with an example embodiment. The dual handgrip measurement device is configured to be held by both the first and second hands of a user and to detect a variety of forces imparted upon the dual handgrip measurement devices including, for example, grip forces applied by the left and right hands, a linear force along a longitudinal axis 12 that extends through the dual handgrip measurement device, such as a compressive force or tensile force, and/or a rotational force applied by the left and right hands of the user about, for example, the longitudinal axis. As such, the dual handgrip measurement device is configured to capture information relating to the forces applied by both the left and right hands of the user and, in one example embodiment, is configured to capture the grip forces applied by both the left and right hands of the user, while concurrently capturing additional information regarding other forces applied by the left and right hands of the user, such as in instances in which the dual handgrip measurement device is held by both the left and right hands of the user and additional forces in the form of a linear force or a rotational force are applied by the left and right hands of the user. Consequently, the dual handgrip measurement device permits information to be more efficiently and expeditiously collected and permits additional types of information to be collected in order to facilitate, for example, a more detailed evaluation of the diagnosis, rehabilitation or other treatment of the user.

As shown in Figure 1, the dual handgrip measurement device 10 includes first and second handgrips 14 configured to be gripped by the left and right hands of the user, respectively. The left and right handgrips are structurally joined together and, in some embodiments, define an integral structure. Alternatively, the first and second handgrips may be separate structures. In some embodiments in which the first and second handgrips are separate structures, the first and second handgrips are mechanically joined to one another, such as by bolts or other types of mechanical fasteners. In other embodiments, however, the first and second handgrips may remain separate, but may both be mounted to a common platform.

In the illustrated embodiment, the first and second handgrips 14 each have a predefined contour or shape. In this regard, each handgrip includes opposed inner and outer portions 16, 18 and a medial portion 20 between the inner and outer portions and recessed relative to the inner and outer portions. The medial portion is configured to be grasped by a respective hand of the user. In some embodiments, the medial portion has a cylindrical or other rounded shape and is sized to be grasped by a hand of the user such that the user's fingers wrap about the majority of the medial portion without wrapping so far about the medial portion so as to contact the hand of the user. The spacing between the inner and outer portions of the handgrip defines the length of the medial portion. In an example embodiment, the length of the medial portion is sized such that the hand of the user will fit between the inner and outer portions and, in some embodiments, will fit snugly between the inner and outer portions.

The inner and outer portions 16, 18 are defined relative to the user's hand with the inner portion positioned interior of the user's hand so as to be proximate the thumb of the user's hand and the outer portion positioned exterior of the user's hand so as to be proximate the smallest finger of the user's hand. The inner and outer portions may have the same size and shape or may have different sizes and/or shapes. Regardless, the inner and outer portions are larger, such as in terms of radius or other cross-sectional dimensions, than the medial portion 20. In an example embodiment, the inner and outer portions are sized to be at least as large as the hand of the user wrapped about the medial portion. Although the inner and outer portions may have different shapes, the inner and outer portions of an example embodiment have a generally cylindrical shape and in this example embodiment, the diameter of the cylindrically shaped inner and outer portions may be at least as large as the cross-sectional size of the hand of the user wrapped about the medial portion.

As shown in Figure 1, the handgrip 14 may define a transition between the inner and outer portions 16, 18 and the medial portion 20. In the illustrated embodiment, a tapered transition is provided. However, the transition between medial portion and the inner and outer portions may be more abrupt in other embodiments with the inner and outer portions extending outwardly, such as in an orthogonal direction, from the medial portion.

The first and second handgrips 14 are configured to be used together in at least some instances. In some embodiments, the first and second handgrips are physically separate from one another, but are configured such that the first handgrip is held and engaged by the left hand of the user concurrent with the second handgrip being held and engaged by the right hand of the user. In the embodiment depicted in Figure 1, however, the first and second handgrips are structurally joined together with the inner portion of the first handgrip structurally joined to the inner portion of the second handgrip. In the embodiment depicted in Figure 1, the first and second handgrips are structurally joined to one another in an aligned relationship, that is, with the medial portions 20 of the first and second handgrips that are to be gripped by the first and second hands of the user aligned with one another. Thus, the first and second handgrips of this example embodiment define a longitudinal axis 12 therethrough. In this example embodiment, the longitudinal axis defines the central axis of the first and second handgrips and, in particular, the central axis of the medial portions of the first and second handgrips.

More generally, however, the longitudinal axis 12 extends through the dual handgrip measurement device 10 and the tensile force, rotational force and/or other forces are applied by a user's hands relative to the longitudinal axis. While the first and second handgrips 14 may be linearly aligned as illustrated and described herein so as define the longitudinal axis as the central axis that extends therethrough, the first and second handgrips of other embodiments are not linearly aligned since the dual handgrip measurement device may have other structural configurations, such as a bent, angled or curved configuration in order to have a more ergonomic layout that conforms with the natural angles of the left and right hands. In still other embodiments, the dual handgrip measurement device may have a wishbone shape, a handle-bar shape, etc. In these embodiments in which the first and second handgrips are not linearly aligned, the longitudinal axis extends through the dual handgrip measurement device, such as through the center of the left and right hands of the user as they grip the dual handgrip measurement device and the forces are applied by the user relative to the longitudinal axis, even though the longitudinal axis is not necessarily defined by the central axis through the first and second handgrips.

In use, a user may grip the first and second handgrips 14 with their left and right hands, respectively, as shown in Figure 2. The user may then apply grip forces with their left and/or right hand to the first and/or second handgrip by squeezing the medial portion(s) 20 of the respective handgrip(s). In some instances, the user may provide other forces, such as a compressive force 42, a tensile, that is, a stretching, force 44, a rotational force 46 about the longitudinal axis 12 or other types of forces based upon interaction between the forces applied by the left and right hands of the user. In order to detect and measure the respective forces applied by the left and/or right hands of the user, the dual handgrip measurement device 10 includes means, such as one or more force sensors, for separately detecting different types of forces.

In this regard, the dual handgrip measurement device 10 includes means, such as first and second force sensors 22, 24 associated with the first and second handgrips 14, respectively, for separately detecting grip forces applied to the first and second handgrips. In addition to detecting the presence or absence of a grip force, the first and second force sensors are configured to measure the grip forces. The first and second force sensors may be embodied in a variety of different manners, but are comprised of electromechanical film (EMFI) or polyvinylidenefluoride (PVDF) sensor materials in one embodiment. Although the first and second force sensors may be embodied by or otherwise associated with the medial portions 20 of the first and second handgrips, respectively, so as to be directly actuated by the grip forces applied by the left and right hands of the user, the first and second force sensors of one embodiment are carried by the inner and outer portions 16, 18 of the respective handgrips so as to be proximate to, but not directly contacted by the user's hand gripping the respective handgrip, thereby avoiding noise or other signal artifacts introduced in an instance in which the force sensor underlies and is directly actuated by the user's hand. As such, the first force sensor of this example embodiment includes a first inner force sensor 22a and a first outer force sensor 22b, while the second force sensor of this example embodiment includes a second inner force sensor 24a and a second outer force sensor 24b.

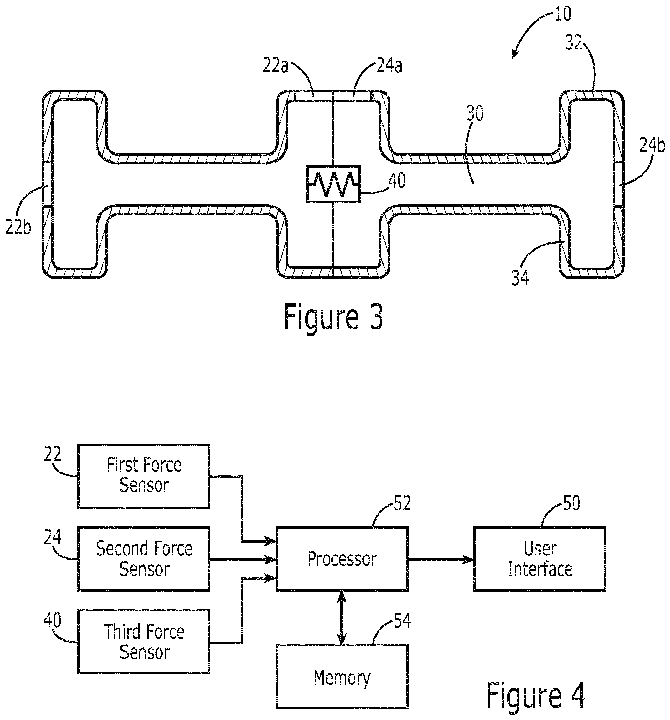

In one embodiment depicted in Figure 3, each handgrip 14 includes a base handgrip structure 30 that defines the relative shapes and sizes of the inner and outer portions 16, 18 and the medial portion 20 of the respective handgrip. The base handgrip structure is generally configured to withstand the application of forces by the user's hands without deformation and, as such, may be formed of a metal, plastic or composite material. Each handgrip of this example embodiment includes a pliable surface covering 32, such as formed of plastic or biomaterials, that envelopes and surrounds the base handgrip structure and that generally conforms to the size and shape of the handgrip structure. In this example embodiment, each handgrip includes a water or a gel, such as a polymer-based hydrogel, or other pliable substance 34 that fills the space between the base handgrip structure and the surface covering. As such, each handgrip of this example defines a predefined volume between the surface covering and the base handgrip structure that is filled with a substance having a predefined density.

Although the surface covering 32 conforms to the shape and size of the handgrip structure 30 in the absence of external forces, the grip force is applied by the user's hand causes displacement of the pliable substance 34 such that a greater percent of the pliable substance moves from the medial portion 20 of the handgrip 14 to the inner and outer portions 16, 18 of the handgrip. The quantity of the pliable substance that moves from a medial portion of the handgrip to the inner and outer portions of the handgrip is representative of the magnitude of the grip force. Thus, the inner force sensors 22a, 24a and the outer force sensors 22b, 24b carried by the inner and outer portions, respectively, of the handgrip of this embodiment are configured to detect pressure imparted upon the respective force sensors by the increased volume of the pliable substance forced into the inner and outer portions by the grip force. Thus, as an increased volume of the pliable substance is displaced from the medial portion to the inner and outer portions of the handgrip, the first and second force sensors 22, 24 are configured to detect the presence of increased pressure. Thus, the first and second force sensors are configured to detect the grip force imparted by the user's hand upon the respective handgrip and are configured to measure the grip force as represented by variations in the pressure imparted upon the respective force sensors by displacement of the pliable substance from the medial portion of the respective handgrip to the inner and outer portions of the respective handgrip. In addition to measuring the grip force applied by the user to a respective handgrip, the use of both of the first and second handgrips permits the grip forces applied to the first and second handgrips by the left and right hands, respectively, to be measured concurrently and permits the difference between the grip forces applied by the left and right hands of the user to be readily determined.

The dual handgrip measurement device 10 includes means for detecting at least one of a linear force along the longitudinal axis 12 defined by the first and second handgrips 14 or a rotational force 46 between the first and second handgrips 14. As described below, the linear force may be detected and measured by the first and second force sensors 22, 24 and/or by a third force sensor 40 configured to interact with each of the first and second handgrips.

In relation to the application of a linear force and/or a rotational force 46, the user concurrently applies forces to both the first and second handgrips 14. In regards to the application of a linear force along the longitudinal axis 12, the user may apply a tensile, that is, a stretching, force 44 in which one or both hands of the user applies a force in an outward direction toward the outer portion 18 of the dual handgrip measurement device 10. For example as shown in Figure 2, in instances in which the user concurrently grips the first and second handgrips with their left and right hands, respectively, a tensile force may be applied by a user attempting to move the left and right hands apart from one another while continuing to maintain their grip upon the medial portions 20 of the respective handgrips. Alternatively, the user may apply a compressive force 42 in which a force is applied in an inward direction toward the inner portion 16 of a respective handgrip. By way of example in which the user grasps the first and second handgrips with their left and right hands, respectively, a compressive force is applied by attempting to force their left and right hands toward one another while maintaining their grip upon the medial portions of the respective handgrips. As an example of a rotational force, the rotational force 46 about the longitudinal axis applied to one or both of the first and second handgrips is detected and measured. In instances in which the user concurrently grasps the medial portions of the first and second handgrips with their left and right hands, respectively, the rotational force of one embodiment is applied as the user attempts to rotate one handgrip about the longitudinal axis without correspondingly rotating the other handgrip and, in some instances, while attempting to rotate the other handgrip in the opposite rotational direction about the longitudinal axis. However, the dual handgrip measurement device may permit the user to apply other types of rotational forces, such as rotational forces in which the user attempts to bend the dual handgrip measurement device. In each of the foregoing examples relating to the application of linear force along the longitudinal axis or rotational force between the first and second handgrips, the placement of the user's hand(s) upon the respective handgrips does not vary, that is, the user's hand does not move or slide inward or outward or rotationally, but, instead, the user applies the respective force while maintaining their grip upon the medial portion of the respective handgrip.

As noted above, in some embodiments, the first and second force sensors 22, 24 are configured to not only detect grip forces, but to also detect the application of a linear force, either a compressive force 42 or a tensile, that is, a stretching, force 44. For example, the first and second force sensors may be configured to detect and measure grip forces that are applied during a first period of time in the absence of a linear force, and to detect and measure linear forces that are applied during a second period of time in the absence of grip forces. In an instance in which one of the grip forces or the linear force has previously been measured, the first and second force sensors may be configured to detect the concurrent application of grip and linear forces and to obtain separate measurements for the concurrently applied grip and linear forces. The forces applied during the concurrent application of grip and linear forces generally differ from the forces applied during the application of grip forces alone since muscles used to concurrently apply grip and linear forces differ from the muscles used to apply grip forces alone and the coordination required to concurrently apply grip and linear forces differs from the coordination required to apply grip forces alone due to the brain level integration of the motor functions for producing the different hand and arm movements. In this situation, the previously measured force value F₁ may serve as the measurement for the type of force that was previously measured, while the difference between the force F₂ that was measured during the concurrent application of grip and linear forces and the previously measured force value F₁ may serve as the measurement for the other type of force that was not previously measured. For example, in an instance in which the grip force was previously measured, the measurement of the linear force may be derived from the measurement of the concurrent application of grip and linear forces by subtracting the previously measured grip force therefrom.

In an embodiment in which the first and second force sensors 22, 24 are utilized to detect the application of a linear force, a compressive force 42 may be detected and measured as a result of displacement of the pliable substance 34 in an inward direction toward the inner portion 16 of a respective handgrip 14, thereby resulting in increased pressure detected by the first and second inner force sensors 22a, 24a. Conversely, a tensile force 44 may be detected and measured as a result of displacement of the pliable substance in an outward direction toward the outer portion 18 of a respective handgrip, thereby resulting in increased pressure detected by the first and second outer force sensors 22b, 24b.

In other embodiments that utilize a third force sensor 40 to detect linear and/or rotational forces, either instead of or in addition to the first and second force sensors 22, 24, the third force sensor may be embodied in various manners. In one embodiment, the third force sensor is embodied by one or more force sensors that bridge between and serve to structurally interconnect the first and second handgrips 14. In relation to the third force sensor configured to detect and measure the application of linear force along the longitudinal axis 12, the third force sensor may be embodied by a string sensor, spring loaded force sensor, hydraulic force sensor, a pneumatic force sensor or the like that extends or is oriented along the longitudinal axis so as to detect and measure the application of the linear force along the longitudinal axis, as shown in Figure 3. Additionally or alternatively, the third force sensor configured to measure or to detect the rotational forces imparted between the first and second handgrips may be embodied by the same or a different sensor than that which detects the application of a linear force. In this regard, the third force sensor that is configured to detect rotational forces may be embodied by a string sensor, spring loaded force sensor, hydraulic force sensor, a pneumatic force sensor or the like that extends or is oriented relative to the longitudinal axis so as to detect and measure the application of the rotational force 46 about the longitudinal axis.

By employing first and second handgrips 14 and a plurality of force sensors 22, 24, 40 configured to measure grip forces applied to the first and second handgrips as well as at least one of a linear force or a rotational force, the dual handgrip measurement device 10 is configured to measure grip forces in a more efficient manner by permitting the grip forces applied by both the left and right hands of the user to be measured concurrently. Additionally, the dual handgrip measurement device provides for a measurement of additional types of forces applied by the left and right hands of the user, thereby providing additional information that can be analyzed with respect to the health condition of the user.

As shown in Figure 4, the dual handgrip measurement device 10 may include or be in communication with a user interface 50 that is configured to provide information to the user regarding their interaction with the dual handgrip measurement device. For example, the user interface of an example embodiment is configured to provide instructions to the user regarding the sequence of interactions between the user and the dual handgrip measurement device. Additionally or alternatively, the user interface of an example embodiment is configured to provide feedback to the user regarding their interaction with the dual handgrip measurement device, such as by providing feedback indicative of the completion of a particular type of interaction with the dual handgrip measurement device. For example, the user interface may initially instruct the user to grip the first and second handgrips 14 with their left and right hands, respectively. Following measurement of the grip forces applied by the left and right hands of the user by the first and second force sensors 22, 24, the user interface may instruct the user to apply a tensile force 44 to the dual handgrip measurement device by attempting to pull their left and right hands apart from one another while maintaining their grip upon the first and second handgrips, respectively. Thereafter, the user interface may instruct the user to terminate the tensile force and to, instead, apply a compressive force 42 by urging their left and right hands towards one another while maintaining their grip upon the first and second handgrips, respectively. Finally, the user interface of this example embodiment may instruct the user to terminate the compressive force and to thereafter apply a rotational force 46 by first attempting to rotate their right hand about the longitudinal axis 12 in an upward direction and their left hand about the longitudinal axis in a downward direction prior to subsequently reversing the direction of rotation such that their left hand is urged to rotate about the longitudinal axis in an upward direction and the right hand is urged to rotate about the longitudinal axis in a downward direction. In other embodiments, the user interface is configured to alter the rhythm with which the instructions are provided and/or may provide different instructions for right and left hands of the user in order to test the user.

The user interface 50 may be embodied in various manners. In one example embodiment, the user interface is associated with and integrated into the at least one of the first and second handgrips 14 or an intermediate structure that is positioned between and serves to structurally interconnect the first and second handgrips. In this regard, the user interface may include one or more buttons designated, for example, grip force, stretching force, compressive force, rotational force 1 and rotational force 2, that may be selectively illuminated to provide instructions to the user. Additionally or alternatively, the user interface may include a display screen carried by the respective handgrip, such as the inner portion 16 of one or both handgrips to provide instructions, e.g., textual and/or graphical instructions, to the user. Additionally or alternatively, the user interface may include one or more speakers carried by at least one of the first and second handgrips so as to provide audible instructions to the user. As such, users with special requirements, such as users with limited eyesight, can utilize the dual handgrip measurement device and be guided through the desired test procedure. By providing instructions visually and/or audibly, the user interface is configured to automatically guide the user in performing the test procedure, thereby increasing the likelihood that the user can properly perform the test procedure in an unsupervised manner, such as at home. In some embodiments, the user interface provides encouragement and/or information to the user during the test procedure.

Although the user interface 50 may be integrated with the first and second handgrips 14 as described above, the user interface of another example embodiment may be separate from the first and second handgrips, while providing instructions regarding interaction by the user with the dual handgrip measurement device 10. In this alternative embodiment, the user interface may be embodied by the user interface of a mobile telephone, a smart watch, a personal fitness device or other type of computing device.

As shown in Figure 4, the dual handgrip measurement device 10 of an example embodiment includes at least one processor 52. The at least one processor may be embodied by at least one of the first and second handgrips 14 or an intermediate structure or may be separate therefrom. In embodiments in which the processor is separate from the first and second handgrips, the processor may be embodied by a mobile telephone, a smart watch, a personal fitness device or other type of computing device.

The processor 52 may be embodied in a number of different ways. For example, the processor may be embodied as one or more of various hardware processing means such as a coprocessor, a microprocessor, a controller, a digital signal processor (DSP), a processing element with or without an accompanying DSP, or various other circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor may include one or more processing cores configured to perform independently. A multi-core processor may enable multiprocessing within a single physical package. Additionally or alternatively, the processor may include one or more processors configured in tandem via the bus to enable independent execution of instructions, pipelining and/or multithreading.

In an example embodiment, the processor 52 may be configured to execute instructions stored in a memory device 54 or otherwise accessible to the processor. Alternatively or additionally, the processor may be configured to execute hard coded functionality. As such, whether configured by hardware or software methods, or by a combination thereof, the processor may represent an entity (e.g., physically embodied in circuitry) capable of performing operations according to an embodiment of the present disclosure while configured accordingly. Thus, for example, when the processor is embodied as an ASIC, FPGA or the like, the processor may be specifically configured hardware for conducting the operations described herein. Alternatively, as another example, when the processor is embodied as an executor of instructions, the instructions may specifically configure the processor to perform the algorithms and/or operations described herein when the instructions are executed. However, in some cases, the processor may be a processor of a specific device (e.g., an image processing system) configured to employ an embodiment of the present invention by further configuration of the processor by instructions for performing the algorithms and/or operations described herein. The processor may include, among other things, a clock, an arithmetic logic unit (ALU) and logic gates configured to support operation of the processor.

Regardless of the manner in which the processor 52 is embodied, the processor is configured to direct the user interface 50 to provide the instructions to the user regarding the user's interaction with the first and second handgrips 14. The processor is in communication with the force sensors, such as the first and second force sensors 22, 24 and the third force sensor 40, so as to receive information from the force sensors representative of a measurement of the force detected the respective force sensors. Thus, the processor is configured to receive information regarding the measurements of the grip forces of the left and right hands of the user by the first and second force sensors. Based upon the information regarding the measurement of the grip forces, the processor is configured to perform various analyses, such as by determining the difference between the grip forces detected by the first and second force sensors, determining the diminution of the grip forces over time, etc. The information provided to the processor by the first and second force sensors may provide an indication as to the timing at which the grip forces were applied. Thus, in an instance in which an instruction is provided to the user to apply grip forces to the first and second handgrips, the processor may be in communication with the user interface and, in some embodiments, may direct the user interface to provide the instructions to the user such that the processor is capable of determining the time at which the instructions to apply grip forces were provided to the user. Based upon the time at which the measurements of the grip forces were obtained by the first and second force sensors, the processor of this example embodiment is configured to determine the lag time between the time at which the instructions to apply the grip forces were provided to the user and the time at which the user actually applied the grip forces.

The processor 52 of this example embodiment is configured to receive information regarding linear forces applied along the longitudinal axis 12 and/or rotational forces 46 about the longitudinal axis as measured by the first and second force sensors 22, 24 or by the third force sensor 40. In instances in which grip forces and a linear force are concurrently applied, the processor of an example embodiment is configured to obtain separate measurements for the grip forces and the linear force, such as based upon prior measurements of one of either the grip forces or the linear force as described above. As with the grip forces, the processor of an example embodiment is configured to determine the time at which the user was instructed to apply one or more of the linear force and/or the rotational force and the time at which the user actually applied the instructed force so as to determine the lag time based upon the difference therebetween.

As shown in Figure 4, the dual handgrip measurement device 10 of an example embodiment includes at least one memory device 54 configured to store representations of the forces detected by the first and second force sensors 22, 24 and, in some embodiment, the third force sensor 40. Based upon the stored representations of the forces detected by the force sensors, the processor 52 of an example embodiment may be configured to determine the manner in which the forces change over time, such as by detecting instances in which the grip forces decrease over time which may be indicative of a change in the health condition of the user as described below. In an example embodiment, the memory device is configured to store additional parameters determined by the processor 52, such as the difference between the grip forces detected by the first and second force sensors and/or the lag time between the time at which the user was instructed to apply a force and the time at which the force was actually applied and measured. In some embodiments, the memory device, such as the same memory device that stores the representation of the forces detected by the force sensors or a different memory, stores instructions that upon execution, such as by the processor, causes the processor to direct the user interface 50 to provide instructions to the user in order to walk the user through the operation of the dual handgrip measurement device, such as by initially instructing the user to grasp the first and second handgrips 14 and then to sequentially apply the different forces of interest.

The memory device 54 may be non-transitory and may include, for example, one or more volatile and/or non-volatile memories. In other words, for example, the memory device may be an electronic storage device (e.g., a computer readable storage medium) comprising gates configured to store data (e.g., bits) that may be retrievable by a machine (e.g., a computing device like the processor). The memory device may be configured to store information, data, content, applications, instructions, or the like for enabling the dual handgrip measurement device 10 to carry out various functions in accordance with an example embodiment. For example, the memory device could be configured to buffer input data for processing by the processor 52. Additionally or alternatively, the memory device could be configured to store instructions for execution by the processor.

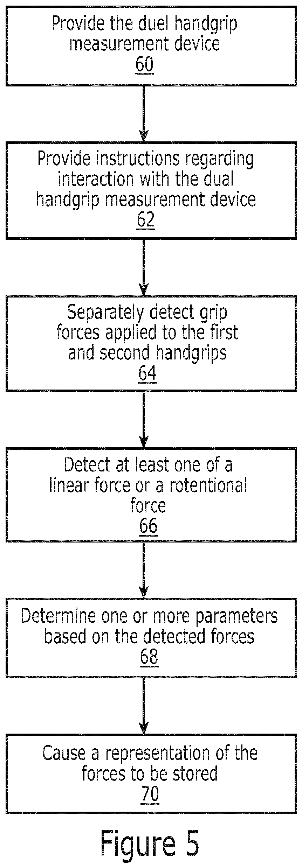

Referring now to Figure 5, a flowchart illustrating operations performed in order to measure grip forces of a user utilizing a dual handgrip measurement device 10 is provided. After initially providing the dual handgrip measurement device having the first and second handgrips 14 configured to be gripped by the left or right hands of the user, respectively, grip forces applied to the first and second handgrips are separately detected by first and second force sensors 22, 24 associated with each of the first and second handgrips, respectively. See blocks 60 and 64 of Figure 5. In some embodiments, the forces may be applied in response to instructions provided via the user interface 50. See block 62. At least one of a linear force along the longitudinal axis 12, such as a tensile force 44 or compressive force 42, or a rotational force 46 between the first and second handgrips is detected, such as by the first and second force sensors and/or by a third force sensor 40. See block 66.

As noted above in conjunction with block 62, in order to guide the use of the dual handgrip measurement device 10 by the user, information may be provided to the user via the user interface 50 regarding interaction by the user with the dual handgrip measurement device. This information may include instructions to the user regarding their interaction with the dual handgrip measurement device. For example, the user may be instructed to grasp the first and second handgrips 14 with their left and right hands, respectively, and to then apply grip forces with their left and right hands. The user may then be instructed to sequentially apply a tensile force 44, a compressive force 42 and a rotational force 46. In some embodiments, the efficiency with which the various forces are measured may be enhanced by detecting the grip forces with the first and second force sensors 22, 24 concurrent with the detection of at least one of the linear force or the rotational force, such as by the first and second sensors or by the third force sensor 40.

While the various forces are applied by the user, the apparatus, such as the processor 52 is configured to receive signals from the respective force sensors as shown in Figure 4. As shown in block 68 of Figure 5, the method of an example embodiment includes determining various parameters based upon the detected forces, such as by determining the various forces applied by the user, a difference between the grip forces detected by the first and second force sensors 22, 24, or the like. Such determinations may be performed by the processor 52 as described above. In this regard, the processor of this example embodiment is configured to process the signals received from the force sensors and to determine the forces applied by the user, such as grip forces based upon signals provided by the first and second force sensors, a compressive force 42 based upon signals provided by the first and second force sensors or by the third force sensor 40, a tensile force 44 based upon signals provided by the first and second force sensors or by the third force sensor, and/or a rotational force 46 about the longitudinal axis 12 based upon signals provided by the third force sensor. Further, a representation of forces detected by the force sensors and the parameters that were determined based upon the detected forces may be caused to be stored, such as within the memory 54. See block 70 of Figure 5. For example, the forces that are determined by the processor may then be stored, such as by the memory, along with an indication of the user, such as a name and/or identifier associated with the user, and a time stamp indicating the day and time at which the forces were recorded.

Based upon the measurement of the forces, such as the grip forces or linear forces applied along the longitudinal axis 12 and/or rotational forces 46 applied about the longitudinal axis, the health condition of the user may be evaluated. In this regard, the grip forces and other forces applied by the left and right hands of the user may provide information regarding a variety of different health conditions of the user. For example, grip forces and other forces applied the left and right hands of a user may be utilized in conjunction with the rehabilitation of the user, such as the rehabilitation from an injury, to determine if and the rate at which the strength of the user is returning.

Alternatively, the grip forces and other forces applied by the left and right hands of the user may be utilized to evaluate the severity of a medical condition and decide upon the mode of treatment. Carpal tunnel syndrome is an example of a musculoskeletal disorder about which grip forces provide diagnostic information. Carpal tunnel syndrome is caused by pressure on the median nerve. The median nerve runs from the forearm through a passageway in the wrist, that is, the carpal tunnel, to the hand. The median nerve provides sensation to the palm side of the thumb and fingers, except for the little finger. The median nerve also provides nerve signals to move the muscles around the base of the thumb, thereby providing for motor function. Repetitive movements of the wrist may lead to tendonitis, that is, irritation of the arm muscle tendons that form a cuff in the wrist. Due to the tendonitis, the space for the median nerve in the carpal tunnel may be limited, resulting in dysfunction of the median nerve. This dysfunction may cause pain, tingling in the wrist and fingers and a decreased grip force. As such, the measurement of the grip force applied by the user and the detection of a decrease over time in the grip force may provide information relevant to a diagnosis of carpal tunnel syndrome. The diagnosis of carpal tunnel syndrome may be relevant in various scenarios including occupational health and ergonomics, sports medicine and music medicine.

Another musculoskeletal disorder which causes the grip force applied by a user to decrease is epicondylitis. With epicondylitis, upper limb and elbow joint irritation causes impingement of tendons and the ulnar nerve. Epicondylitis may cause a decrease in the grip force that a user is capable of applying. Similarly, a frozen shoulder can decrease the grip force applied by a user. As such, the measurement of the grip force applied by the user and the detection of a decrease over time in the grip force may provide information relevant to a diagnosis of epicondylitis or a frozen shoulder. The measurement of grip forces may not only facilitate diagnosis of carpal tunnel syndrome, epicondylitis or other musculoskeletal disorders, but may also provide information that may be considered in relation to the treatment that is prescribed for the musculoskeletal disorder, such as physiotherapy, kinesiotape, local pain cream, oral medication, orthopaedics and the like, such as by determining whether the grip forces are increasing following treatment of the patient. More generally, the dual handgrip measurement device 10 may provide biofeedback for a patient undergoing training or rehabilitation.

The measurement of grip forces also facilitates the diagnosis of peripheral nerve damage and the determination of treatment options for the peripheral nerve damage. In this regard, sensory motor damage may occur in polyneuropathies, such as due to diabetes or a renal or autoimmune disease, which results in a reduction in the grip forces applied by the user as a result of a decrease in hand sensing or nerve motor function. As such, the measurement of the grip strength provides information regarding the severity of the underlying disease by identifying a change in the grip force applied by a user in the early stages of sensory motor damage, thereby permitting the disease to be treated to prevent further impairment in hand function. The measurement of grip forces and the detection of a reduction in the grip forces may also permit joint diseases, such as a hand, wrist and elbow arthrosis, such as due to aging, as well as rheumatoid arthritis to be detected. By measuring the grip forces applied by the user, treatments, such as physiotherapy, may also be tailored to the individual.

A measurement of the grip forces also assists in the identification of brain diseases, such as a brain stroke or brain damage in the sensory or motor areas of the brain. In this regard, brain damage to the left sensory motor cortex causes a decrease in the grip forces applied by the right hand, while brain damage to the right sensory motor cortex causes a decrease in the grip forces applied by the left hand. Additionally, different types of bimanual functions are affected as the healthy side of the brain attempts to compensate for the damaged side. By measuring the grip forces as well as linear and/or rotational forces, the level of decreased hand and upper arm functions may be objectively clinically evaluated. In some instances, handgrip is utilized as a part of neurorehabilitation. Even in instances of a relatively mild stroke, the minor changes in hand function often remain undetected such that use of the dual handgrip measurement device 10 permits even these more minor changes in hand functions to be detected by the reduction in handgrip such that appropriate rehabilitation or other treatment may be pursued.

As another example, patients with an occasional cardiac arrhythmia burst may have an increased risk of a brain vascular embolism. These embolisms cause a transient ischemic attack (TIA) of the brain. However, the cardiac arrhythmia may have passed and the clinical signs of TIA may have abated by the time that the patient is evaluated medically. However, the measurement of the grip forces of the patient may indicate that the patient is having increased difficulty in applying the same level of grip forces as in the past and may serve to facilitate diagnosis or at least further investigation of the cardiac arrhythmia burst.

Additionally, grip forces and linear and/or rotational forces applied by the hands of a user may be utilized to identify, particularly the early stages, of brain diseases, such as memory disorders, Alzheimer's disease, Parkinson's disease or the like. A conventional measurement of grip force by a handheld dynamometer may produce results from which it is difficult to determine if the grip force has diminished due to a brain disease or simply due to aging. By instructing a user to utilize the dual handgrip measurement device in order to apply a number of different forces, such as grip forces and linear and/or rotational forces in a sequence, information indicative of a brain disease may be gathered for a user having difficulty in switching from the application of one type of force to another, such as evidenced by an increased lag in switching between the application of different types of force. By repeating different sequences of interactions with the dual handgrip measurement device 10, the severity level of a brain disease, its progress and/or the efficacy of the medical treatment may be evaluated.

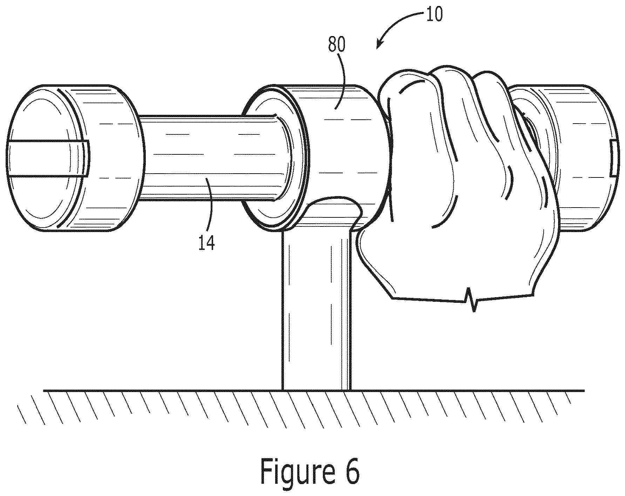

The dual handgrip measurement device 10 of an example embodiment is multi-functional in that the dual handgrip measurement device may be utilized in a variety of different configurations. As described above, the dual handgrip measurement device includes first and second handgrips 14 configured to be gripped by the left and right hands of a user, respectively. However, in some instances, the dual handgrip measurement device is only intended to be utilized by one hand. As shown in Figure 6, the dual handgrip measurement device of this example embodiment may be held in a fixture 80 that serves to fix the position and orientation of the dual handgrip measurement device. The user may grip one of the first and second handgrips with a respective hand and then apply the various forces, such as a grip force, a linear force, a rotational force or the like.

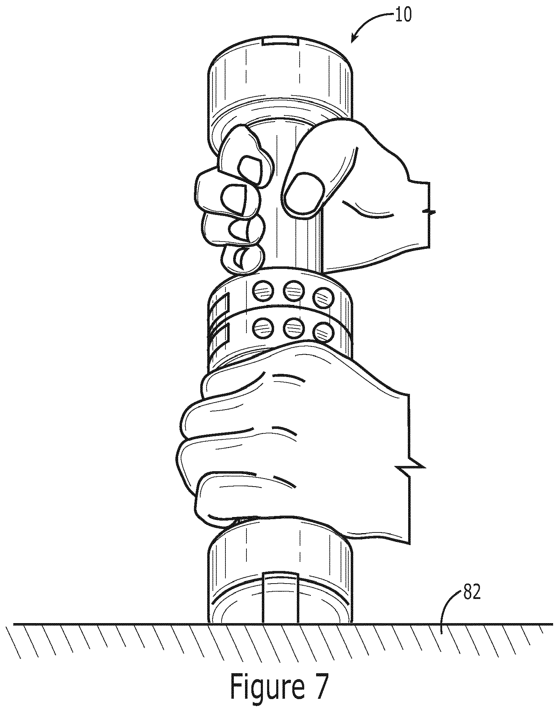

Still further, the dual handgrip measurement device 10 may be placed upright on a work surface 82 as shown in Figure 7 such that the outer portion of one of the handgrips contacts the work surface and the longitudinal axis 12 extends substantially vertically. In this embodiment, one or both of the first and second handgrips 14 may be gripped by the user and one or more forces may then be applied. In this embodiment, the forces that are applied may include a force intending to push the dual handgrip measurement device toward the work surface that supports the dual handgrip measurement device. As such, additional types of forces may be measured and utilized, such as in conjunction with the evaluation of a health condition of the user. Regardless, the multi-functional nature of the dual handgrip measurement device permits the collection of a variety of different forces from which the health condition of the user may be evaluated in a more fulsome manner.

As described above, Figure 5 is a flowchart of an apparatus 10, method, and computer program product according to example embodiments of the invention. It will be understood that each block of the flowchart, and combinations of blocks in the flowchart, may be implemented by various means, such as hardware, firmware, processor, circuitry, and/or other devices associated with execution of software including one or more computer program instructions. For example, one or more of the procedures described above may be embodied by computer program instructions. In this regard, the computer program instructions which embody the procedures described above may be stored by a memory device 54 of an apparatus employing an embodiment of the present invention and executed by a processor 52 of the apparatus. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may be stored in a computer-readable memory that may direct a computer or other programmable apparatus to function in a particular manner, such that the instructions stored in the computer-readable memory produce an article of manufacture, the execution of which implements the function specified in the flowchart blocks. The computer program instructions may be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

A computer program product is therefore defined in those instances in which the computer program instructions, such as computer-readable program code portions, are stored by at least one non-transitory computer-readable storage medium with the computer program instructions, such as the computer-readable program code portions, being configured, upon execution, to perform the functions described above, such as in conjunction with the flowchart of Figure 4. In other embodiments, the computer program instructions, such as the computer-readable program code portions, need not be stored or otherwise embodied by a non-transitory computer-readable storage medium, but may, instead, be embodied by a transitory medium with the computer program instructions, such as the computer-readable program code portions, still being configured, upon execution, to perform the functions described above.

Accordingly, blocks of the flowchart support combinations of means for performing the specified functions and combinations of operations for performing the specified functions for performing the specified functions. It will be understood that one or more blocks of the flowchart, and combinations of blocks in the flowchart, may be implemented by special purpose hardware-based computer systems which perform the specified functions, or combinations of special purpose hardware and computer instructions.

In some embodiments, certain ones of the operations above may be modified or further amplified. Furthermore, in some embodiments, additional optional operations may be included, such as illustrated by the blocks having dashed outlines in Figure 5. Modifications, additions, or amplifications to the operations above may be performed in any order and in any combination.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A dual handgrip measurement device comprising:
first and second handgrips configured to be gripped by left and right hands of a user, respectively; and
first and second force sensors associated with and configured to separately detect grip forces applied to the first and second handgrips, respectively,
wherein the dual handgrip measurement device is configured to detect at least one of a linear force along a longitudinal axis extending through the dual handgrip measurement device or a rotational force between the first and second handgrips.

2. A dual handgrip measurement device according to Claim 1 further comprising a third force sensor configured to detect the linear force including at least one of a compressive force or a tensile force along the longitudinal axis.

3. A dual handgrip measurement device according to Claim 1 or Claim 2 wherein the first and second force sensors are configured to detect the grip forces concurrent with detection of at least one of the linear force or the rotational force by the third force sensor.

4. A dual handgrip measurement device according to any one of Claims 1 to 3 wherein the first and second handgrips are structurally joined together.

5. A dual handgrip measurement device according to any one of Claims 1 to 4 wherein at least one of the first or second force sensors comprises an inner force sensor located interior of and an outer force sensor located exterior of a respective hand.

6. A dual handgrip measurement device according to any one of Claims 1 to 5 further comprising a user interface configured to provide information to the user regarding interaction with the dual handgrip measurement device.

7. A dual handgrip measurement device according to any one of Claims 1 to 5 further comprising a user interface configured to provide instructions to the user regarding a sequence of interactions between the user and the dual handgrip measurement device.

8. A dual handgrip measurement device according to any one of Claims 1 to 7 further comprising at least one processor configured to determine a difference between the grip forces detected by the first and second force sensors.

9. A method of measuring grip forces of a user utilizing a dual handgrip measurement device, wherein the method comprises:
providing the dual handgrip measurement device comprising first and second handgrips configured to be gripped by left and right hands of a user, respectively;
separately detecting grip forces applied to the first and second handgrips with first and second force sensors associated with the first and second handgrips, respectively; and
detecting at least one of a linear force along a longitudinal axis extending through the dual handgrip measurement device or a rotational force between the first and second handgrips.

10. A method according to Claim 9 wherein detecting the linear force comprises detecting at least one of a compressive force or a tensile force along the longitudinal axis.

11. A method according to Claim 9 or Claim 10 further comprising providing information to the user via a user interface of the dual handgrip measurement device regarding interaction by the user with the dual handgrip measurement device.

12. A method according to any one of Claims 9 to 11 wherein providing information comprises providing instructions to the user regarding a sequence of interactions between the user and the dual handgrip measurement device.

13. A method according to any one of Claims 9 to 12 wherein the grip forces are detected by the first and second force sensors concurrent with detection of at least one of the linear force or the rotational force.

14. A method according to any one of Claims 9 to 13 further comprising determining a difference between the grip forces detected by the first and second force sensors.

15. A method according to any one of Claims 9 to 14 further comprising causing a representation of forces detected by the first and second force sensors to be stored.
